# EUROPEAN PATENT APPLICATION

(11) **EP 1 163 953 A2**
(43) Date of publication of application: **19.12.2001**
(21) Application number: 01304485.4
(22) Date of filing: 22.05.2001
(51) Int. Cl.: B01J 8/18, B01J 8/22, C07C 51/25, C07C 53/08, C07C 253/24, C07C 253/26, B01J 19/00

(54) **Apparatus and process for oxidation reactions**

(30) Priority: 14.06.2000 GB 0014580
(71) Applicant: BP Chemicals Limited, London EC2M 7BA (GB)
(72) Inventor: Bristow, Timothy Crispin, Hull, HU12 8DS (GB); Clarke, Robert William, Hull, HU12 8DS (GB); Williams, Bruce Leo, Hull, HU12 8DS (GB); Colman, Derek Alan, Sunbury on Thames Middlesex, TW16 7LN (GB); Reid, Ian Allan Beattie, Sunbury on Thames Middlesex, TW16 7LN (GB); Newton, David, Sunbury on Thames Middlesex, TW16 7LN (GB); Becker, Stanley John, Addlestone, Surrey KT15 2LP (GB)
(74) Representative: Perkins, Nicholas David

(57) **Abstract**

A reactor and process for using same, for containing a solid catalyst for heterogeneous gas-phase reactions into which reactor there extends at least one inlet pipe for a molecular oxygen-containing gas which has means for surrounding a substantial portion of the pipe in the reactor with an inert fluid and optionally also has means for suppressing ingress to the inlet pipe from the reactor of flame, reagents, products, catalyst or combinations thereof. The reactor and process are particularly suitable for fluid bed reactions.

## Description

The present invention relates in general to apparatus for introducing a molecular oxygen-containing gas into a reactor containing a solid catalyst for a heterogeneous gas-phase reaction and to processes in which a molecular oxygen-containing gas is introduced into a reactor containing a solid catalyst for a heterogeneous gas-phase reaction.

Reactors and their use in processes involving a molecular oxygen-containing gas with a solid catalyst for heterogeneous gas phase reactions are known.

Fluid bed reactors and their use in processes involving a molecular oxygen-containing gas with a solid catalyst for a heterogeneous gas phase reaction are also known.

For example, EP-A-0685449 discloses a process for manufacturing vinyl acetate in a fluid bed reactor comprising feeding ethylene and acetic acid into the fluid bed reactor through one or more inlets, feeding an oxygen-containing gas into the fluid bed reactor through at least one further inlet, co-joining the oxygen-containing gas, ethylene and acetic acid in the fluid bed reactor while in contact with a fluid bed catalyst material to enable the ethylene, acetic acid and oxygen to react to produce vinyl acetate and recovering the vinyl acetate from the fluid bed reactor. According to EP-A-0685449, the oxygen may be added in pure form or as an admixture with inert gas such as nitrogen or carbon dioxide. Since the oxygen and hydrocarbons are not mixed until they are both inside the reactor, catalyst is present when they meet and reaction proceeds immediately, causing the oxygen partial pressure to drop. Thus, an advantage of feeding an oxygen-containing gas to the reactor through at least one further inlet in addition to the ethylene and acetic acid reactants is that it allows significantly higher levels of oxygen to be safely employed without a high inventory of flammable gas mixtures.

Also, EP-A-0546677 discloses a process for oxidising ethane to acetic acid in a fluidized bed reaction zone. In the example illustrated in EP-A-0546677, ethane is joined with a recycle stream containing water, CO, CO₂, O₂, ethylene and ethane and the combined stream is fed to the fluid bed reactor. A molecular oxygen-containing stream and steam are introduced separately into the fluid bed reactor. The hot oxidation products exit the top of the reactor.

WO 01/03823 published after the priority date of the present application, relates to a sparger for oxygen injection into a fluid bed reactor which includes a conduit for conducting an oxygen feed, a nozzle connected to the conduit for passage of the oxygen feed from the conduit to the outside of the sparger, the nozzle including an orifice and a shroud, and insulation (such as ceramic insulation) surrounding the conduit and also the shroud substantially the full length of the shroud. The apparatus may be used for the production of acrylonitrile via propane ammoxidation.

The use of fluid bed reactors for heterogeneous gas-phase reactions involving molecular oxygen-containing gas enables high concentrations of oxygen in the molecular oxygen-containing gas to be used. Such high concentrations of oxygen can present safety risks, for example if the supply pipe fractures, especially within the reactor.

The need for apparatus for the safe introduction of a molecular oxygen-containing gas into a reactor containing a catalyst for a heterogeneous gas-phase reaction is not limited to fluid bed reactors, such molecular oxygen-containing gases may also be introduced into fixed bed reactors. There is thus a need for apparatus for the safe introduction of a molecular oxygen-containing gas into a reactor containing a solid catalyst for a heterogeneous gas-phase reaction.

According to the present invention there is provided a reactor for containing a solid catalyst for a heterogeneous gas-phase reaction into which reactor there extends at least one inlet pipe for a molecular oxygen-containing gas, in which, said inlet pipe has means for surrounding a substantial portion of said pipe in said reactor with an inert fluid.

According to the present invention there is also provided a process in which a molecular oxygen-containing gas is introduced into a reactor containing a solid catalyst for a heterogeneous gas-phase reaction in which said molecular oxygen-containing gas is introduced into said reactor through at least one inlet pipe extending into said reactor, said inlet pipe having means which surrounds a substantial portion of said pipe in said reactor with an inert fluid.

In a preferred embodiment of the present invention, the inlet pipe further has means for suppressing ingress to the inlet pipe from the reactor of flame, reagents, products, catalyst or combinations thereof.

The present invention provides a solution to the need with at least one inlet pipe for a molecular oxygen-containing gas which has means for surrounding a substantial portion of said pipe in said reactor with an inert fluid and optionally also has means for suppressing ingress to the inlet pipe from the reactor of flame, reagents, products, catalyst or combinations thereof.

By inert is meant that the inert fluid is substantially resistant to reaction with the molecular oxygen-containing gas and/or other reactants in the reactor. Typically, at least 85 % of the inlet pipe in the reactor is surrounded by the means for surrounding the pipe with the inert fluid.

The inert fluid surrounding a substantial portion of the at least one inlet pipe in the reactor may comprise an inert gas, for example selected from the group consisting of nitrogen, carbon dioxide, helium, argon, neon, krypton and mixtures thereof. Small amounts of oxygen may also be present in the inert fluid provided that it does not present any hazard.

The means for surrounding a substantial portion of the inlet pipe in the reactor with inert fluid may comprise an outer pipe surrounding a substantial portion of one or more inlet pipes for molecular oxygen containing gas in the reactor and provided with a supply of inert fluid. The outer pipe may also have an advantage of providing structural support to the inlet pipe which may thus be smaller than otherwise and so have a reduced molecular oxygen-containing gas inventory. This improves safety. A further advantage is that the outer pipe may also provide thermal insulation to the molecular-oxygen containing inlet pipe thus reducing the potential for reagents in the reactor to condense on the inlet pipe. The outer pipe may be concentric with the inlet pipe or may be any suitable shape to surround a substantial portion of one or more inlet pipes. Preferably, the inlet pipe extends into the reactor by only a small amount of its length beyond the means for surrounding the inlet pipe with inert fluid, for example sufficient only to achieve a suitable weld joint.

Means for allowing for differential expansion of the inlet pipe and the means for surrounding the pipe with inert fluid may be provided. Such differential expansion means may include bends in the inlet pipe and/or pig-tails.

Preferably, the inert fluid surrounding the portion of the inlet pipe in the reactor is at a different pressure to that of the molecular oxygen-containing gas in the inlet pipe. In this case, if the inlet pipe breaks and/or leaks, this may be detected by detecting a flow of gas either to or from the inert fluid surrounding the inlet pipe. Thus, if the inert fluid is at a higher pressure than the molecular oxygen-containing gas, in the event of a break or leak of the inlet pipe, a drop in pressure of the inert fluid may be detected (if the inert fluid is supplied from a limited or sealed source) and/or the presence of inert fluid for example in the reactor gaseous effluent may be detected. Such a higher pressure also reduces the possibility of oxygen-containing gas leaking into the supply of inert gas (which has a high inventory and so would present a hazard). Also, the use of a higher pressure of inert fluid than the pressure of the oxygen-containing gas means that if there is a pipe fracture the escape of inert fluid dilutes the oxygen-containing gas and improves safety. Conversely, if the inert fluid is at a lower pressure than the molecular oxygen-containing gas, a break or leak of the inlet pipe may be detected by a rise in pressure in the inert fluid (if the inert fluid is supplied from a sealed source) and/or by detection of molecular oxygen-containing gas in the inert fluid surrounding the inlet pipe. The inert fluid surrounding the inlet pipe may be sealed so that a breakage or leak of the inlet pipe may be detected by a pressure change of the inert fluid. Alternatively, a limited supply of inert fluid may be provided sufficient to replenish minor leaks but insufficient in the event of a major failure, which would be detectable by a pressure drop in the inert fluid.

Thus, the reactor may further comprise one or more of (a) means for detecting a change in pressure of said inert fluid surrounding said inlet pipe, (b) means for detecting the presence of inert fluid in gaseous effluent from said reactor and (c) means for detecting molecular oxygen-containing gas in said inert fluid surrounding said inlet pipe. One or more of these detector means may be operably connected to means for automatically shutting off the supply of oxygen-containing gas if an unsafe situation arises - for example if more than a certain number of inlet nozzles become blocked and/or loss of the inert fluid surrounding the inlet pipe(s).

Suitably, the pressure of molecular oxygen-containing gas in the inlet pipe is in the range 100 kPa to 10 MPa. Suitably, the difference between the pressure of inert fluid substantially surrounding the inlet pipe and the molecular oxygen-containing gas is in the range 1 kPa to 10 MPa. Preferably, the inert fluid is at a pressure greater than the pressure of the molecular oxygen-containing gas.

The means for suppressing ingress to the inlet pipe from the reactor of flame, reagents, products, catalyst or combinations thereof may comprise the provision of the molecular oxygen-containing gas in the inlet pipe at a higher pressure than the pressure in the reactor, to prevent back-flow which could cause an explosion. The pressure of the gas in the inlet pipe may be sufficiently high to prevent ingress even in the event of an explosion of an oxygen-containing gas bubble in the reactor adjacent the inlet pipe.

The means for suppressing ingress to the inlet pipe from the reactor of flame, reagents, products, catalyst or combinations thereof may comprise the provision to the outlet of the inlet pipe in the reactor of a restriction for example, comprising one or more orifices. This restriction may reduce or prevent back-flow of flame, reagents, products and catalyst, especially when used in a fluid bed reactor. The restriction may provide a suitable back pressure to suppress ingress yet provide an exit gas velocity which does not lead to damage to the catalyst. The restriction, such as one or more orifices, is preferably located at a sufficient distance from the outlet of the inlet pipe in the reactor to provide uniform flow out of the inlet pipe. On the other hand, the restriction, such as one or more orifices, is preferably located sufficiently close to the outlet of the inlet pipe in the reactor such that a potential detonation is avoided. As a suitable compromise the restriction, such as one or more orifices, is preferably located, 4 to 5 pipe diameters from the end of the inlet pipe. The restriction, such as one or more orifices, is preferably located within the region of the inlet pipe surrounded by the means for surrounding the inlet pipe with inert fluid. Thus, for example, a fall in pressure of the inert fluid surrounding an inlet pipe may be used to detect a burn back of the inlet pipe to the restriction.

More than one inlet pipe may be provided in the reactor of the present invention. Preferably, these should be located for even distribution of the oxygen-containing gas across the cross-section of the reactor. The inlets should be positioned sufficiently far apart that flame propagation will not occur between them. Preferably, the distance between inlets is significantly in excess of the potential flame length. The potential flame length is determined by factors such as the inlet pipe diameter and inlet gas velocity. The inlets should be positioned and inlet pressures and velocities selected, so that the molecular oxygen-containing gas is dispersed and mixed in the region of the inlet. The inlets should be positioned not too close to the reactor walls, for example to avoid poor gas distribution and for safety in the unlikely event there is a shock wave following a detonation. The inlets should be positioned so that the molecular oxygen-containing gas does not impinge directly on surfaces or other structures in the reactor such as inlets for other reactants.

The molecular oxygen-containing gas for the inlet pipes may be provided from a common source such as a common end box having a low inventory and optionally provided with a safety purge during shut-down. Molecular oxygen-containing gas and other gases may also be introduced to the reactor by other inlets not according to the present invention, for example as components in recycle gases and/or mixed feed gases. An advantage of the present invention is that it provides a safe means of introducing a molecular oxygen-containing gas which has a high oxygen concentration.

Suitable molecular oxygen-containing gases for use in the present invention include air, oxygen-enriched air and oxygen gas with minor amounts of impurities such as nitrogen, carbon dioxide, argon etc. The concentration of impurities is preferably not greater than 0.4 % by volume. The concentration of oxygen in the molecular oxygen-containing gas is suitably in the range of greater than 20 % by volume, preferably in the range 30 to 100 % by volume.

The inlet pipe is adapted to be operably connected to a supply of molecular oxygen-containing gas. The supply of molecular oxygen-containing gas may be provided through one or more flow restriction means, for example one or more orifices, which restrict the flow of molecular oxygen-containing gas to the inlet pipe. This has the advantage of restricting the flow in the event that the inlet pipe in the reactor becomes significantly damaged and/or other inlets become blocked.

In a fluid bed reactor, the at least one inlet pipe is preferably located such that the molecular oxygen-containing gas is introduced directly into the fluidized catalyst bed, rather than below it. Preferably, the inlet is directed downwards in the bed which has an advantage of reducing ingress of solids when the bed is not operational.

Preferably, the reactor is a fluid bed reactor. Fluid bed reactors have advantages that come from the introduction of the molecular oxygen-containing gas separately from the other reactants. An advantage of the present invention is that it provides improved safety when the molecular oxygen-containing gas is introduced directly into the fluid bed catalyst of a fluid bed reactor.

Suitable processes and in particular fluid bed processes, for use of the present invention include (a) the acetoxylation of olefins, for example the reaction of ethylene, acetic acid and oxygen to produce vinyl acetate, (b) the oxidation of ethylene to acetic acid and/or the oxidation of ethane to ethylene and/or acetic acid, (c) the ammoxidation of propylene, propane or mixtures thereof to acrylonitrile and (d) the oxidation of C4's to maleic anhydride.

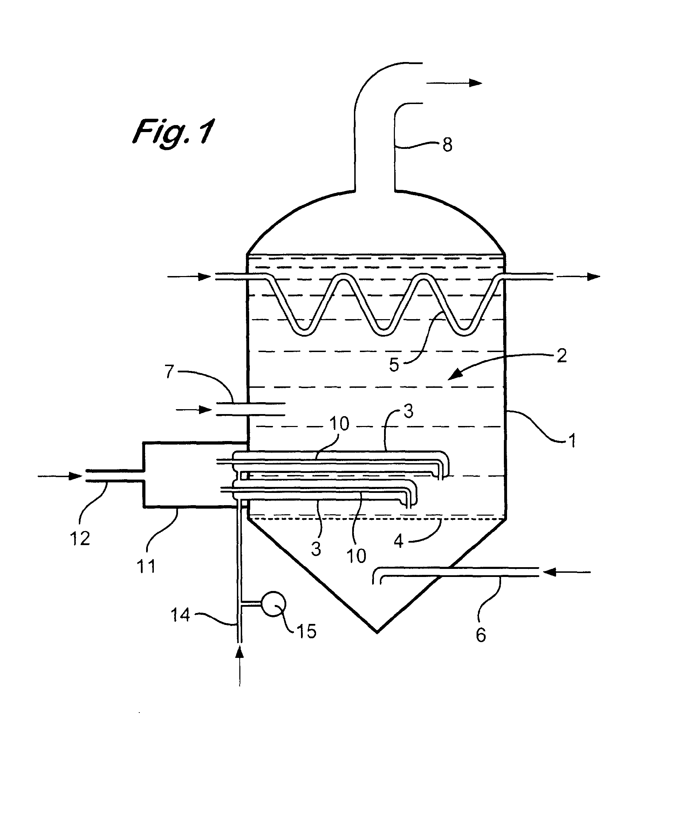

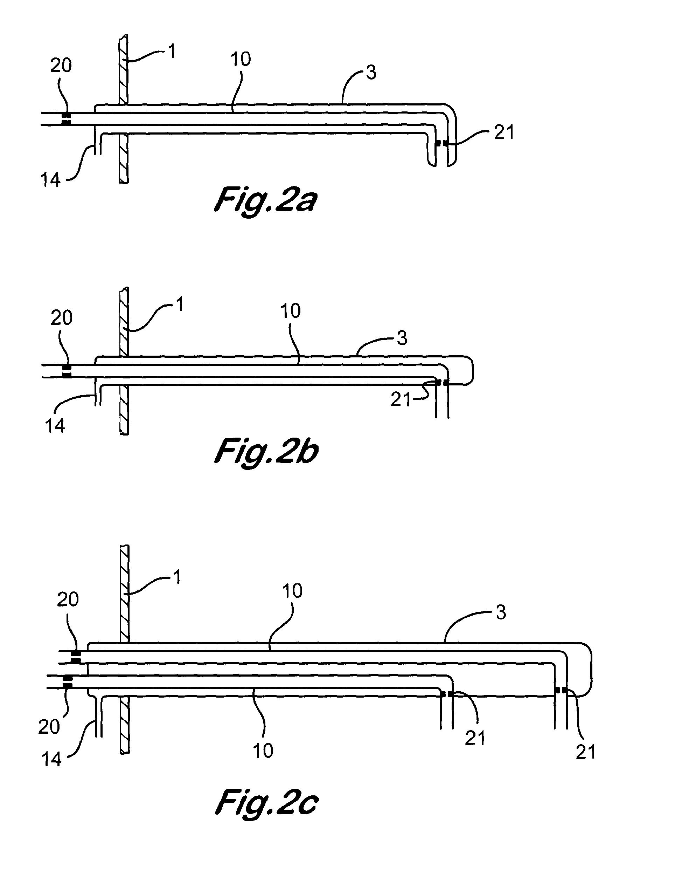

The invention will now be illustrated by way of example only and with reference to the drawings in which Figure 1 represents in schematic form, a cross-section of a fluid bed reactor according to the present invention and Figure 2 represents in schematic form, a cross-section of three designs of inlet pipe according to the present invention.

Referring to Figure 1. A reactor (1) for a fluid bed reaction such as the acetoxylation of ethylene to vinyl acetate contains in use, a fluidized bed of catalyst (2), for example a palladium/gold catalyst supported on a silica support supported on a suitable grid (4). The reactor (1) is provided with at least one inlet pipe (10) for a molecular oxygen-containing gas. The fluid bed reactor (1) is also provided with cooling coils (5) and a supply of fluidising gas comprising recycle gases, ethylene reactant and optionally oxygen reactant through inlet (6). A supply of acetic acid reactant is also provided through inlet (7). The cooling coils (5) may be used to heat up the reactor at start-up, being provided with a supply of hot fluid.

Each inlet pipe (10) is connected at one end to a low inventory common end box (11), which in turn is connected to a supply of molecular oxygen-containing gas (12) and optionally to a shut-down safety purge (not shown). Each inlet pipe extends into the reactor (1) and a substantial portion of the inlet pipe in the reactor is surrounded by an outer pipe (3) which is connected to a supply of inert gas such as nitrogen (14). In use, the pressure of molecular oxygen-containing gas in the inlet pipes (10) is greater than the pressure in the reactor (1) and the pressure of the inert gas in the outer pipes (3) is at a pressure greater than that of the molecular oxygen-containing gas in the inlet pipes (10). The supply of inert gas is provided with means (15) for detecting a change in pressure in the event that the inlet pipes (10) leak or break.

Figure 2 shows in schematic cross-section three designs of inlet pipe according to the present invention and such as are shown in Figure 1. A substantial portion of each inlet pipe (10) within the reactor (1) is surrounded by an outer pipe (3) which is connected to a supply of inert gas such as nitrogen. Each inlet pipe (10) is provided with an orifice plate (20) to limit the supply of molecular oxygen-containing gas and an orifice plate (21) near its outlet to reduce or prevent ingress of flame, reagents, products and fluid bed catalyst. Suitably the orifice plate has more than one orifice, for example 3 orifices in a triangular pitch and provides sufficient back-pressure to suppress ingress of flame, reagents, products and fluid bed catalyst but with an exit linear velocity of gas which would not lead to excessive attrition of the fluid bed catalyst. In one design (Figure 2a), the inlet pipe (10) extends concentrically from the outer pipe (3) by only a short distance. In the second design (Figure 2b) the inlet pipe (10) extends radially from the outer pipe (3) by only a short distance. More than one inlet pipe may be surrounded by a common outer pipe as is shown in Figure 2c.

The apparatus of Figures 1 and 2 may be used in processes involving the use of molecular oxygen-containing gas such as the acetoxylation of ethylene to produce vinyl acetate. In use, ethylene reactant and recycle gases are passed through inlet (6) to fluidise the catalyst bed (2) in the reactor (1). Acetic acid reactant, preferably as a liquid, is introduced into the fluidised bed through inlet (7). A molecular oxygen-containing gas is introduced into the fluidised catalyst bed through at least one inlet pipe (10). Heat of reaction is removed at least in part by the cooling coils (5) provided with a supply of cooling water. The gaseous reaction products are removed from outlet (8).

Inert gas such as nitrogen is supplied to the outer pipes (3) surrounding a substantial portion of the inlet pipes in the reactor (1) at a pressure greater than the pressure of the molecular oxygen-containing gas in the inlet pipes (10). In the event that the inlet pipes break or leak this may be detected as a change in pressure by detector (15) and appropriate operational action may be taken.

Similar apparatus may be used for other reactions involving the use of molecular oxygen-containing gas - for example the oxidation of ethylene to acetic acid and/or the oxidation of ethane to ethylene and/or acetic acid, the ammoxidation of propane, propylene or mixtures thereof to produce acrylonitrile and the oxidation of C4's to maleic anhydride.

## Claims

1. A reactor for containing a solid catalyst for a heterogeneous gas-phase reaction into which reactor there extends at least one inlet pipe for a molecular oxygen-containing gas, in which, said inlet pipe has means for surrounding a substantial portion of said pipe in said reactor with an inert fluid.

2. A reactor as claimed in claim 1 in which at least 85 % of the said pipe in said reactor is surrounded by said surround means.

3. A reactor as claimed in claim 1 or claim 2 in which said inert fluid comprises an inert gas.

4. A reactor as claimed in claim 3 in which said inert gas is selected from the group consisting of nitrogen, carbon dioxide, helium, argon, neon, krypton and mixtures thereof.

5. A reactor as claimed in any one of the preceding claims in which said means for surrounding a substantial portion of said inlet pipe in said reactor with inert fluid comprises an outer pipe surrounding a substantial portion of one or more inlet pipes for molecular oxygen containing gas in said reactor and provided with a supply of inert fluid.

6. A reactor as claimed in any one of the preceding claims which further comprises means for allowing for differential expansion of said inlet pipe and said means for surrounding said pipe with inert fluid.

7. A reactor as claimed in any one of the preceding claims which further comprises one or more of (a) means for detecting a change in pressure of said inert fluid surrounding said inlet pipe, (b) means for detecting the presence of inert fluid in gaseous effluent from said reactor and (c) means for detecting molecular oxygen-containing gas in said inert fluid surrounding said inlet pipe.

8. A reactor as claimed in any one of the preceding claims in which said inlet pipe further has means for suppressing ingress to the inlet pipe from the reactor of flame, reagents, products, catalyst or combinations thereof.

9. A reactor as claimed in claim 8 in which said ingress suppression means comprises means for providing molecular oxygen-containing gas in said inlet pipe at a higher pressure than the pressure in said reactor.

10. A reactor as claimed in any one of claims 8 to 9 in which said ingress suppression means comprises a restriction to the outlet of said inlet pipe.

11. A reactor as claimed in claim 10 in which said restriction comprises one or more orifices.

12. A reactor as claimed in claim 10 or claim 11 in which said restriction is located at a distance from the outlet of said inlet pipe in the reactor such that a potential detonation is avoided.

13. A reactor as claimed in claim 10 or claim 11 in which said restriction is located 4 to 5 pipe diameters from the end of the inlet pipe.

14. A reactor as claimed in any one of claims 10 to 13 in which said restriction is located within the region of said inlet pipe surrounded by said means for surrounding said inlet pipe with inert fluid.

15. A reactor as claimed in any one of the preceding claims having more than one inlet pipe.

16. A reactor as claimed in claim 15 in which the distance between inlets is significantly in excess of the potential flame length.

17. A reactor as claimed in claim 15 or claim 16 in which said molecular oxygen-containing gas for said inlet pipes is provided from a common end box having a low inventory and optionally provided with a safety purge during shut-down.

18. A reactor as claimed in any one of the preceding claims in which said inlet pipe is adapted to be operably connected to a supply of molecular oxygen-containing gas provided through one or more flow restriction means which restrict the flow of molecular oxygen-containing gas to the inlet pipe.

19. A reactor as claimed in any one of the preceding claims in which the reactor is a fluid bed reactor.

20. The use of a reactor as claimed in any one of the preceding claims in a process selected from the group consisting of the acetoxylation of olefins, the reaction of ethylene, acetic acid and oxygen to produce vinyl acetate, the oxidation of ethylene to acetic acid, the oxidation of ethane to ethylene and/or acetic acid, the ammoxidation of propylene, propane or mixtures thereof to acrylonitrile and the oxidation of C4's to maleic anhydride.

21. A process in which a molecular oxygen-containing gas is introduced into a reactor containing a solid catalyst for a heterogeneous gas-phase reaction in which said molecular oxygen-containing gas is introduced into said reactor through at least one inlet pipe extending into said reactor, said inlet pipe having means which surrounds a substantial portion of said pipe in said reactor with an inert fluid.

22. A process as claimed in claim 21 in which at least 85 % of the said pipe in said reactor is surrounded with said inert fluid.

23. A process as claimed in claim 21 or claim 22 in which said inert fluid comprises an inert gas.

24. A process as claimed in claim 23 in which said inert gas is selected from the group consisting of nitrogen, carbon dioxide, helium, argon, neon, krypton and mixtures thereof.

25. A process as claimed in any one of claims 21 to 24 in which said reactor comprises a reactor as claimed in any one of claims 5 to 19.

26. A process as claimed in any one of claims 21 to 25 in which there is a difference in pressure between the inert fluid substantially surrounding the inlet pipe and the molecular oxygen-containing gas in the range 1 kPa to 10 MPa.

27. A process as claimed in claim 26 in which the inert fluid is at a pressure greater than the pressure of the molecular oxygen-containing gas.

28. A process as claimed in any one of claims 21 to 27 comprising a process selected from the group consisting of the acetoxylation of olefins, the reaction of ethylene, acetic acid and oxygen to produce vinyl acetate, the oxidation of ethylene to acetic acid, the oxidation of ethane to ethylene and/or acetic acid, the ammoxidation of propylene, propane or mixtures thereof to acrylonitrile and the oxidation of C4's to maleic anhydride.
